# EUROPEAN PATENT APPLICATION

(11) **EP 1 067 179 A1**
(43) Date of publication of application: **10.01.2001**
(21) Application number: 99401745.7
(22) Date of filing: 09.07.1999
(51) Int. Cl.: C12N 7/04, C12N 15/40, C12N 15/45, C12Q 1/70, A61K 39/155, C07K 14/08, C07K 14/155, A61P 31/14

(54) **Method to select attenuated paramyxoviridae useful for vaccines and therapeutics**

(71) Applicant: PIERRE FABRE MEDICAMENT, 92100 Boulogne-Billancourt (FR)
(72) Inventor: Curran, Joseph, 1223 Thonex, Genève (CH); Kolakofsky, Dan, 1205 Genève (CH); Garcin, Dominique, 74140 Chens-sur-Léman (FR); Power, Ultan, 74160 Archamps (FR)
(74) Representative: Ahner, Francis

(57) **Abstract**

The present invention relates to a method using the cellular interferon-mediated antiviral response to select attenuated Paramyxoviridae. The invention also relates to a method to select viral nucleic acid sequence encoding for a mutated non-structural protein that is useful to produce an attenuated virus. The use of attenuated virus to develop vaccine is also described.

## Description

The present invention relates to a method using the cellular interferon-mediated antiviral response to select attenuated Paramyxoviridac. The invention also relates to a method to select viral nucleic acid sequence encoding for a mutated non-structural protein that is useful to produce an attenuated virus. The invention also relates to the use of such attenuated viruses for vaccine applications.

Interferons (IFNs) are a family of cytokines originally identified by their ability to confer cellular resistance against viral infection (14), and which are also involved in cell growth regulation and immune activation (30,33). Infection of cells with a wide variety of viruses directly induces the transcription and synthesis of some IFNs (e.g., IFN-a4 and IFN))(24,34). These IFNs are secreted and interact with constitutively expressed cell surface receptors in an autocrine or paracrine manner, transducing signals via the JAK-STAT pathway to activate >50 IFN-stimulated genes (ISGs), which include the type I IFNs (6). Some of these ISGs are responsible for the versatile biological effect of IFNs, including their anti-viral activity. In addition to establishing an anti-viral state in uninfected cells, the IFN system also helps eliminate virally infected cells (38). The IFN system is thus thought to be essential for the survival of higher vertebrates, because it provides an early line of defense, days before the onset of the specific immune response (33).

Sendai virus (SeV), a model paramyxovirus routinely used to induce types I IFN in cell culture, is a naturally occurring respiratory pathogen of laboratory mice (15). SeV strains exist which differ markedly in their pathogenicity for mice, but the determinants of their virulence are largely unknown. Our reference SeV strain Z was originally isolated in the early 1950s in Japan, and adapted to grow in embryonated hen's eggs. This adaptation and continuous passage in eggs undoubtedly reduces its virulence for mice, as the LD50 of SeV^{Z} is ca. 6 x 10³, whereas that of the more recently isolated Ohita "field" strain (from a lethal animal house epidemic and which was passaged only in mice (SeV^{M})) is ca. 4 x 10¹ (16). SeV^{M}, however, grows poorly ir cell culture, and its adaptation for growth in LLC-MK2 (monkey kidney cells led to virus clones that exhibited various degrees of reducec pathogenicity for mice. One of these clones, MVC11 (SeV^{MVC}) appeared to be totally avirulent (LD50 of >8 x 10⁵, i.e., none of the mice died a the highest possible dose).

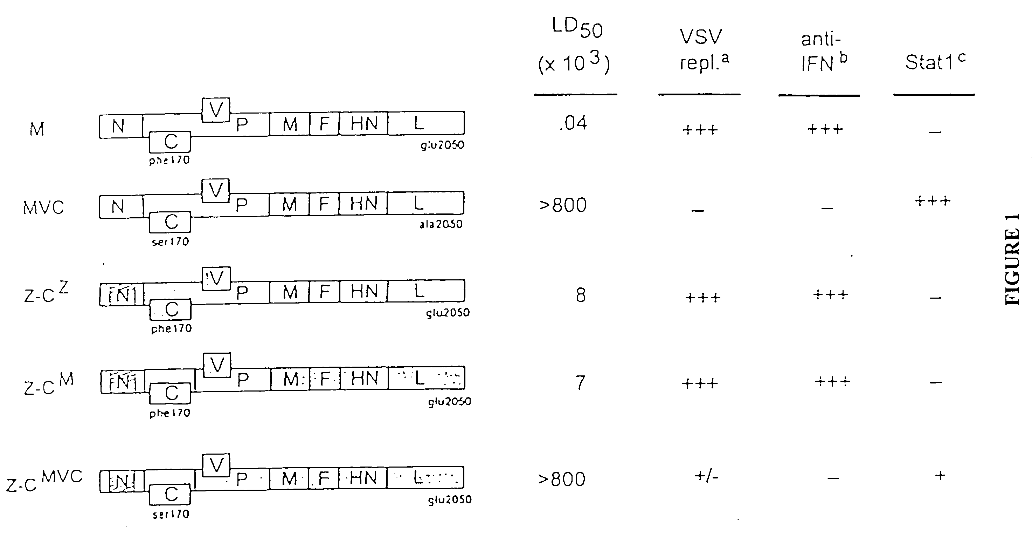

Remarkably, SeV^{MVC} contained only two amino acid substitutions relative to the parental SeV^{M}, namely, F170S in the C proteins, anc E2050A in the L protein (fig. 1) (16). These two mutations presumably account for the relative inability of SeV^{MVC} to replicate in the mouse respiratory tract and cause serious disease. In a previous study, we partially examined which of the two substitutions was responsible for the above phenotypes, by exchanging the C gene of SeV^{Z} with that or itself, SeV^{M}, or SeV^{MVC}, in turn (generating rSeV^{Z}-C^{Z}, rSeV^{Z}-C^{M}, and rSeV^{Z}-C^{MVC}, respectively, fig.1)(12). Wild-type rSeV^{Z}-C^{Z} (recovered from DNA) has the same virulence for mice as the natural Z virus (LD50 ol ca. 6 x 10³), and the replacement of the resident C^{Z} gene with that of strain M did not affect this virulence. Replacement of the resident C^{Z} gene with C^{MVC} in two independently isolated rSeV, however, increased the LD50 of rSeV^{Z}-C^{MVC} by >2 logs, such that they were now as avirulent as SeV^{MVC}. As the only known difference between rSeV^{Z}-C^{M} and rSeV^{Z}-C^{MVC} is the CF^{170S} mutation, the normal function of the C proteins appears to be required for virulence in mice. The wild-type SeV C proteins are also known to act as promoter-specific inhibitors of viral RNA synthesis (1,28,32). The C^{F170S} mutation is similarly associated with the loss of this function, and is at least partially responsible for the enhanced replication of these viruses in MK2 cells.

Although rSeV^{Z}-C^{MVC} was avirulent, it appeared to grow normally in the mouse respiratory tract during the first day of the infection However, whereas virus titers in rSeV^{Z}-C^{M} or rSeV^{Z}-C^{Z}-infected mice lungs increased daily for ca. 5 days, virus titers in rSeV^{Z}-C^{MVC}-infected mice increased only during the first day, and rScV^{Z}-C^{MVC} was then quickly cleared from the lungs (12). A similar early restriction of the mouse infection was found for rSeV which specifically cannot express their AUG¹¹⁴-initiated C protein (22), or which cannot edit their P gene mRNA and therefore do not express their V proteins (7,8,19). The rapidity of the host anti-viral response in immunologically naive mice in limiting the ensuing mutant SeV infections suggested that some aspect of host innate immunity might be involved, presumably due to the loss of V and C protein function. In this view, one function of the SeV C and V proteins would be to modulate the innate immune response, to sustain multiple cycles of virus replication in the mouse respiratory tract (19). In this invention, the inventors report that the SeV C proteins play a role in counteracting the IFN-mediated cellular anti-viral response.

In one aspect, the invention features a method for selecting attenuated virus wherein the genome of said virus contains at least one mutation compared to a wild type virus genome in at least one non-structural protein encoding gene, said method comprising the steps of (1) infecting a eukaryotic host cell with said mutated virus, said host cell being permissive to said virus, (2) determining the host cell anti-viral response mediated by interferon to the viral infection, (3) selecting said virus that induce said interferon mediated response that is higher than the interferon-mediated anti-viral response developed by a host cell infected by said wild-type virus.

In an other aspect, the invention features a method for selecting attenuated virus wherein the genome of said virus contains at least one mutation compared to the wild type virus in at least one non-structural protein encoding gene, said method comprising the steps of (1) transfecting a host cell with said genome, said host cell having all the elements required to replicate and transcribe the mutated genome and to facultatively regenerate viral particles, (2) determining the host cell antiviral response mediated by interferon, (3) selecting said virus whose genome induce said antiviral response that is higher than the interferon-mediated antiviral response developed by a wild-type virus genome.

In an other prefered aspect, the invention features a method for selecting a nucleic acid sequence encoding for a mutated non-structural protein of a virus, wherein said protein is conferring to the virus an attenuated phenotype, said method comprising the steps of (1) transfecting a host cell with said nucleic acid sequence, said host cell having all the elements required to express said non-structural protein, (2) measuring the host cell interferon mediated response, (3) selecting said nucleic acid sequence that induce said response that is higher than the interferon mediated response of a host cell transfected with a wild-type nucleic acid sequence coding the non-structural protein.

For a given virus, wild type means a strain presenting phenotypic features common to the majority of the strains that can be isolated in the nature. A wild-type virus is according to the invention a naturally occuring virus that possess a mechanism for countering or minimizing the host cell IFN-induced antiviral response. An attenuated virus of the invention is a virus that do not possess a mechanism for countering or minimizing the host cell IFN-induced antiviral response as strong as the one of the wild type virus.

Host cell means cell that can be infected by the virus of the invention. A permissive host cell is a cell that allows the virus to multiply and to produce new virions.

As used herein, the terms " nucleic acid sequence " refer to a polynucleotide sequence such as a single or double stranded DNA or RNA sequence, or transcription products obtained from said DNA ; such a polynucleotide sequence has been isolated or synthesised and may be constituted with natural or non natural nucleotides. The term " gene " refers also to the nucleic acid sequence that encodes for a protein or a peptide. The nucleic acid sequence of the invention is facultatively in a vector suitable for expression of nucleic acid. The nucleic acid sequence in a vector can be in host suitable for expressing the nucleic acid sequence. A " vector is a replicon in which another polynucleotide segment is attached, so as to bring the replication and/or expression to the attached segment. The vector can contain genes conferring for exemple either tetracycline, gentamicin, hygromycin or methotrexate resistance for use as selectable markers.

" Elements required for the expression " refers to the polynucleotide sequences which are necessary to effect the expression of coding sequences to which they are ligated. The nature of such control sequences differs depending upon the host organism ; in eukaryotes, generally, such control sequences include promoter, enhancer, polyadenylation sequences and transcription termination sequence. The term " elements required for the expression " is intended to include, at the minimum, all components whose presence is necessary for expression, and may also include additional components whose presence is advantageous. The vectors containing the nucleic acid sequence of interest can be tranferred into the host cell by well-known methods, which vary depending on the type of the cellular host. For exemple, calcium phosphate, electroporation and lipofection may be used. These expression vectors are typically replicable in the host cells either as episomes or as integral part of the host chromosomal DNA.

The invention features a method wherein said virus belongs to the group of negative single strand RNA enveloped virus, said negative single strand RNA enveloped virus is selected in the group consisting of Paramyxoviridae, Orthomyxoviridae, Rhabdoviridae, Bunyaviridae, Arenaviridae

The invention also features a method wherein said Paramyxoviridae is selected in the group consisting of Newcastle disease virus, measles virus, mumps virus, human respiratory syncitial virus (hRSV), bovine respiratory syncytial virus (bRSV), human para-influenza virus types 1, 2, 3, 4a and 4b (h-PIV), bovine para-influenza virus type 3 (b-PIV), hendra virus, canine distemper virus, rinderpest virus, simian virus 5, Sendaï virus.

In a preferred embodiment, the invention features a method wherein said gene is selected in the group consisting of paramyxovirus protein C and C' encoding genes, morbilivirus C gene, rubulavirus NS genes, pneumovirus NS genes, W gene of Newcastle disease virus.

In an other embodiment, the invention relates to a method wherein said Paramyxoviridae protein C encoding gene is selected in the group consisting of human para-influenza virus type 1 C genes, human para-influenza virus type 3 C genes, measles virus C gene, canine distemper virus C gene, rinderpest virus C gene, hendra virus C genes, Sendaï virus C genes. The invention also relates to a method wherein said Paramyxoviridae NS gene is selected in the group consisting of human respiratory syncytial virus NS1 gene, bovine respiratory syncytial virus NS1 gene, mumps virus NS2 gene, simian virus 5 NS2 gene, human para-influenza type 2 NS2 gene, human para-influenza type 4a NS2 gene, human para-influenza type 4b NS2 gene, human respiratory syncytial virus NS2 gene, bovine respiratory syncytial virus NS2 gene.

In an other aspect, the invention features a method wherein said mutation into said gene is a substitution, a deletion or an addition; the mutation of said gene introduces at least one amino-acid change in the non-structural protein. The mutation into the viral genome or in the nucleic acid sequence is selected in a group consisting of naturally occurring mutation, genetically engineered mutation, chemically induced mutation, physically induced mutation. In a prefered embodiment the mutation is induced by a chemical treatment with 5-Fluorouracile. In an other embodiment, the mutation can be induced by a physical treatment such as gamma-, UV- or X-ray irradiation.

Nucleic acid sequence of the invention encoding for a mutated non-structural protein can be prepared by the techniques known in the art. For example, it may include the recombinant DNA techniques and among others, site directed mutagenesis or random mutagenesis of DNA sequence which encodes said non-structural protein or a particular domain or region of said protein.

Site-directed mutagenesis of nucleic acid sequence encoding the non-structural protein of the invention can be used to introduce mutation in the said nucleic acid sequence by methods known in the art. Such methods may, among others, include polymerase chain reaction (PCR) with oligonucleotide primers bearing one or more mutations (Ho *et al.,* 1989 Gene 77 : 51-59) or total synthesis of mutated genes (Hostomsky Z. *et al.* 1989 Biochem. Biophys. Res. Comm 161 :1056-1063). These methods can be used to create variants which include, e.g., deletions, insertions or substitutions of residues of the known amino acids sequence of the non-structural protein of the invention. PCR mutagenesis using reduced Taq polymerase fidelity can also be used to introduce random mutations into a cloned fragment of DNA (Leung *et al.,* 1989 technique 1 :11-15). The pool of amplified DNA fragments are inserted into appropriate cloning and/or expression vectors to provide random mutant librairies. The mutants DNA fragment is then tested in the method of the invention to select the DNA fragment that encodes for a mutated non-structural protein that is confering an attenuated phenotype to the virus. Random mutagenesis can also be performed according to the method of Mayers *et al*.(1985, Science 229 :242). This technique includes generations of mutations, e.g., by chemical treatment or irradiation of single-strand DNA *in vitro,* and synthesis of a complementary DNA strand. The mutation frequency can be modulated by modulating the severity of the treatment, and essentially all possible substitutions can be obtained.

The invention features a method wherein the host cell response mediated by interferon is determined by at least one of the following means (1) measure of the level of transcription of a gene, the transcription of which is inducible by interferon, (2) measure of expression of a protein, the expression of which is inducible by interferon, (3) measure of the viral proliferation. The above gene is composed of (i) a promoter containing at least one functional interferon stimulated response element (IRSE), said promoter being selected in the group consisting of the genes involved in interferon signal transduction pathway, more particularly the IFN-inducible gene 9-27, and (ii) a coding sequence selected in a group consisting of a coding sequence naturally under the control of a promoter according to a) and a sequence encoding for a reporter gene; the promoter being operably linked to the coding sequence.

The methods for measuring the level of transcription of a gene or the level of expression of a protein are well known to the man skilled in the art (sec Sambrook, J., Fritsch, E.F., and Maniatis (1989), T. Sec. Ed. Cold Spring Harbor Lab., Cold Spring Harbor, New York). For exemple, when the reporter gene is the luciferase gene, the level of protein expressed can be measured using "luciferase assay system" (Promega) according to the manufacturers instructions; this kit permits analysis of luciferase activity in cell extract.

A " coding sequence " is a polynucleotide sequence which is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, cDNA, and recombinant polynucleotide sequences.

" Operably linked " refers to a juxtaposition wherein the components so described are in a relationship permitting them to function in their intended manner. A promoter " operably linked " to a coding sequence is ligated in such a way that expression of the coding sequence is achieved under conditions compatible with the promoter.

In an other aspect, the invention features a method wherein said reporter gene is selected in the group consisting of genes coding for enzymes such as luciferase, β-galactosidase, alkaline-phosphatasc, glucose-oxydase and genes coding for luminescent proteins such as the green fluorescent protein (GFP). The " reporter gene " of the invention is coding for a peptide which by its chemical nature, has an analytically identifiable characteristic or provides directly or indirectly an analytically identifiable signal which allows the determination or measure of the level of transcription of a gene or the reporter gene. In the present invention, the level of transcription of a reporter gene encoding for a reporter molecule or the level of expression of a reporter molecule reflects the ability of a non-structural protein of the invention to counteract the IFN-mediated cellular anti-viral response.

Among commonly used peptides encoding by the reporter gene of the invention are enzymes that arc preferably horseradish peroxidase, glucose oxidase, β-galactosidase, alkaline-phosphatase and luciferase, among others. In a preferred embodiment, the luciferase gene is used. The substrate to be used with the specific enzymes are generally chosen for the production, upon hydrolysis by the corresponding enzyme, of a detectable colour change. Substrate can be soluble or insoluble, added into the culture medium or in the organism, or present into the host cell, depending upon the chosen method. For example, 5-bromo-4-chloro-3-indoyl phosphate/nitroblue tetrazolium is suitable for use with alkaline phosphatase conjugates ; for peroxidase conjugates, 1,2-phenylenediamine-5-aminosalicylic acid, 3,3,5,5,-tetramethylbenzidine, tolidine or dianisidine are commonly used.

According to another aspect of the invention, the host cell is selected in a group consisting of a cell that is part of a living animal or a eukaryotic cell that is cultured *in vitro*. In a preferred embodiment, the living animal is a rodent and more preferably a mouse. " host cells " or " cells " denote, for example, insects cells and mammalian cells that can be, or have been, used as recipients for virus, viral genome, recombinant vector, or other transfer nucleic acid sequences and include the progeny of the original cell which has been either infected by the virus or transformed with nucleic acid sequences, viral genome or recombinant vector. Examples for mammalian cells include murine BF cells, human MRC5 cells. Mammalian cells permit the expression of the non-structural protein of the invention in an environment that favours important post-translational modifications such as folding and cysteine pairing, addition of complex carbohydrate structures.

In a preferred embodiment, the invention relates to a method wherein interferon is further added to the culture medium of host cells. While host cell is part of a living animal, interferon can be administrated to the animal by techniques known by a man skilled in the art, preferably by intravenous, intramuscularly or intradermal injection. According to another aspect of the invention, interferon is naturally produced either by other cells of the living animal, or by the host cell of the living animal. In an other preferred embodiment, the host cell is *in vitro* cultured, the interferon being either produced naturally by the host cell, or the interferon being produced by a host cell that has been genetically engineered to produce interferon. According to the invention, the term interferon (IFN) means all types of IFN (i.e. IFNα, IFNβ, IFNγ) and eventually their isotypes.

The invention also features a method wherein said nucleic acid sequence is cloned into a recombinant expression vector that contains all the elements required for the expression of said non-structural protein. The vector can eventually contain the elements necessary for its replication. In a preferred embodiment, the vector is a pTM-1 vector that carries T7 polymerase promoter and an encephalomyocarditis virus internal ribosomal entry site (EMCV IRES). The host cell contains all the elements required to express the nucleic acid sequence of the invention; these elements are either naturally present in the host cell or they are genetically engineered to contain these elements. In a preferred embodiment, an expression vector encoding for a T7-polymerase under the control of a cytomegalovirus (or others) eukaryotic promoter is co-transfected with the pTM-1 vector of the invention to ensure the transcription and the expression of the nucleic acid sequence of the invention.

The invention also relates to a kit for determining the host cell response mediated by interferon comprising at least a nucleic acid construct containing (1) a promoter sequence containing at least one functional interferon stimulated response element (IRSE), said promoter being selected in the group consisting of the genes involved in interferon signal transduction pathway, more particularly the IFN-inducible gene 9-27 ; (2) a coding sequence selected from a group consisting of a coding sequence naturally under the control of a promoter according to a) and a sequence encoding for a reporter gene. In an other embodiment, the invention relates to a kit further comprising a host cell selected in the group consisting of a murine cell, particularly BF cells, and a human cell, particularly MRC5 cells.

The invention features a method to identify a mutation able to confer an attenuated phenotype to a virus wherein said method is comprising the following steps: (1) sequencing said gene encoding said non-structural protein of an attenuated virus selected by the method of the invention, or sequencing said nucleic acid sequence encoding for a mutated non-structural protein selected by the method of the invention, (2) comparing the sequence of a wild type virus to the sequence obtained in (1), and (3) identifying the mutation or the mutations.

The invention further comprises a method for the production of an attenuated virus comprising the step of introducing in the genome of a virus at least one mutation identified by the method of the invention.

In an other aspect, the invention features an attenuated virus obtainable by the method of the invention; the invention also features the use of such an attenuated virus as a drug, particularly as a vaccine for the prophylactic and/or therapeutic treatment of viral infection. In an other aspect, the invention features the use of such an attenuated virus as a delivering vehicle for genes encoding proteins of vaccine interest other than those of the virus itself, particularly to generate bivalent or multivalent vaccines to treat or prevent human or veterinary diseases caused by infectious pathogens, specifically viruses, bacteria and parasites. The invention also features the use of an attenuated virus of the invention as a delivering vehicle for genes encoding proteins of vaccine interest other than those of the virus itself, particularly to generate vaccines to treat or prevent human or veterinary non-infectious diseases, specifically cancer and auto-immune diseases.

The vaccine of the invention is an immunogenic, or otherwise capable of eliciting protection against the virus, composition useful for treatment of an individual ; according to the invention, an individual indicates an animal that is susceptible to infection by the virus, more particularly a member of the Paramyxoviridae, and includes, but is not limited to, dogs, cats, mice, rats, rabbits, sheep, goats, pigs, cattle, , primates, including humans. These vaccines may either be prophylactic (to prevent infection) or therapeutic (to treat disease after infection). Such vaccines comprises the attenuated virus of the invention alone, or in combination with " pharmaceutically acceptable carriers", which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particle. Such carriers are well known to those of ordinary skill in the art. Additionally, such carriers may function as immunostimulating agents also called " adjuvants " ; preferred adjuvants to enhance effectiveness of the composition include, but are not limited to : (1) aluminium salts (alum), such as aluminium hydroxyde, aluminium phosphate, aluminium sulphate, etc.. ; (2) oil-in-water emulsion formulations, such as for example MF59 (WO 90/14 837), SAF, Ribi™ adjuvant system (Ribi Immunochem, Hamilton, MT USA) ; (3) saponin adjuvants ; (4) complete Freunds adjuvant (CFA) and incomplete Freunds adjuvant (IFA) ; (5) cytokines such as interleukines (I1-1, I1-2, etc...), macrophage colony stimulating factor (M-CSF), tumor necrosis factor (TNF) etc... ; (6) other substances that act as stimulating agents to enhance the effectiveness of the composition.

The vaccines are conventionally administered parenterally, e.g., by injection, either subcutaneously or intramuscularly. Additional formulations suitable for other modes of administration include oral, intranasal, and pulmonary formulations, suppositories, and transdermal applications. Oral, intranasal and pulmonary formulations are most preferred for the attenuated paramyxoviruses. Dosage treatment may be single dose schedule or a multiple dose schedule. The vaccine may be administered in conjunction with other immunoregulatory agents.

The invention also features the use of an attenuated virus of the invention as a delivering vehicle for gene therapy.

In an other aspect, the invention features the nucleic acid sequence obtainable by the method of the invention. The invention also relates to the use of such nucleic acid sequence to produce an attenuated virus.

The invention also relates to the non-structural protein encoded by the nucleic acid sequence of the invention.

The figures and exemples presented below arc provided as further guide to the practitioner of ordinary skill in the art and arc not to be construed as limiting the invention in anyway.

**Figure 1 :** The genotypes of the various SeV examined in this study are represented as rectangles on the left, in which the linear array of genes (not drawn to scale) are shown as boxes. The relative positions of the C and V ORFs, which overlap the P ORF, are also indicated. M strain sequences are colored white, Z strain sequences are gray, and H strain sequences are hatched. These rSeV^{z} viruses all carry a tagged N gene, in which this protein migrates differently on SDS-PAGE than that of strain Z, to distinguish these viruses from natural SeV^{z}. The amino acids at positions 170 of the C gene, and 2050 of the L gene, are shown below the genomes. The phenotypes of these virus infections of mice (LD₅₀) are taken from ref. 12 and 16. Those of BF cells; the ability of VSV to replicate following a period of SeV infection (a), to prevent IFN from establishing an antiviral state (b), and the resulting intracellular levels of the Stat1 proteins (c), were determined in this study.

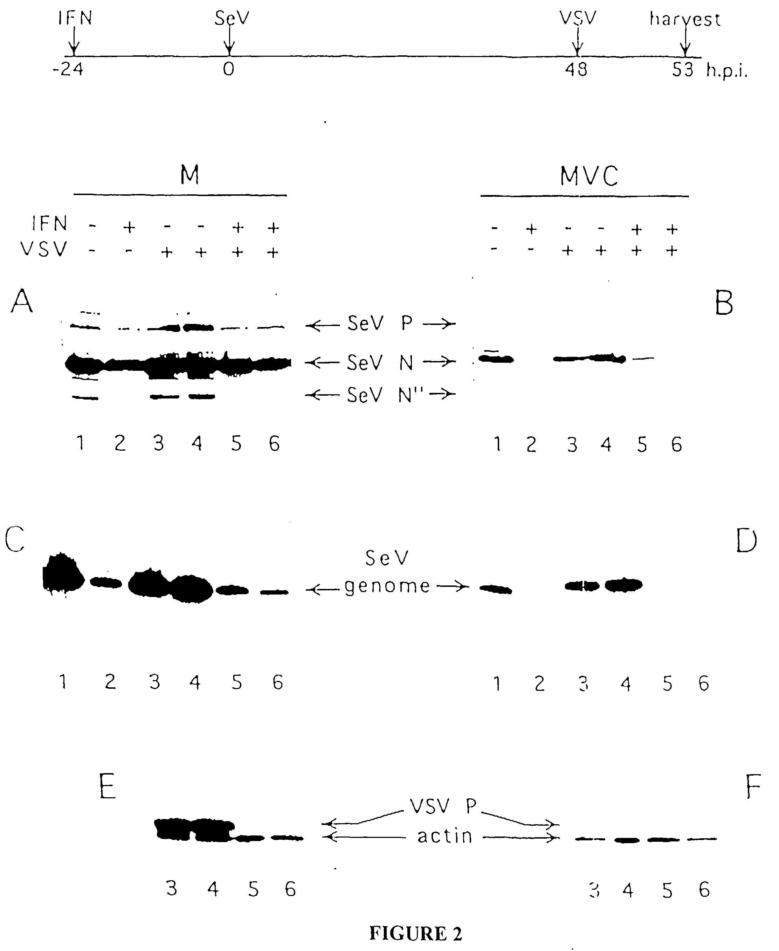

**Figure 2 :** Parallel cultures of BF cells were treated (or not) with 100 U of recombinant IFN-β, infected 24h later with 10-20 pfu/cell of either SeVM or SeVMVC, and then superinfected with 50 pfu/cell of VSV at 48h post-SeV infection, as indicated. Lanes 3,4, and 5,6, represent independent duplicate infections. All cultures were harvested at 53 hpi, cytoplasmic extracts were prepared, and equal samples (2% of 10 cm dish) were; a) separated by SDS-PAGE and immunoblotted with a mixture of anti-SeV P and anti-SeV N antibodies (panels A & B), or a mixture of anti-VSV P and anti-actin antibodies (panels E & F), and b) 10% of a 10 cm dish was used to isolate total RNA and the relative amounts of genome RNA present were determined by primer extension.

SeV N" indicates a natural breakdown product of the N protein. A time line of the experiment is shown above.

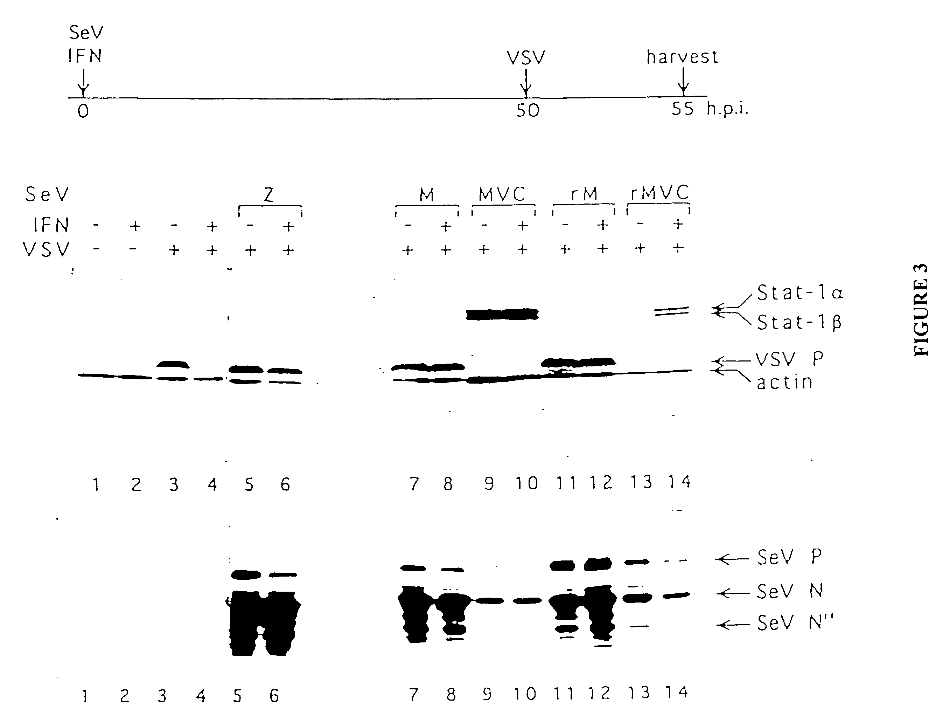

**Figure 3 :** Parallel cultures of BF cells were treated (or not) with 100 U of IFN-β, and infected at the same time with 10-20 pfu/cell of either rSeV^{Z}-C^{Z} (Z), SeV^{M} (M), SeV^{MVC} (MVC), rSeV^{Z}-C^{M} (rM), or rSeV^{Z}-C^{MVC} (rMVC), and then superinfected with 50 pfu/cell of VSV at 50h post-SeV infection, as indicated above. All cultures were harvested at 55 hpi, cytoplasmic extracts were prepared, and equal samples (2% of a dish) were separated by SDS-PAGE and immunoblotted with a mixture of anti-VSV P, anti-Stat1, and anti-actin antibodies (top panel), or a mixture of anti-SeV P and anti-SeV N antibodies (bottom panel). A time line of the experiment is shown above.

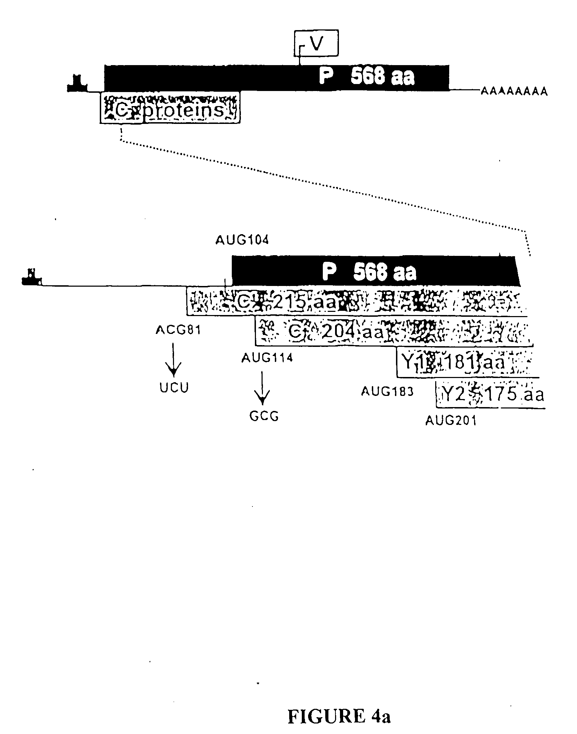

**Figure 4A :** The three ORFs expressed as protein (P, C, and V) are shown as horizontal boxes, drawn roughly to scale (above). A blow-up of the 5' end of the mRNA is shown in the middle, and the five ribosomal start sites in this region are indicated. The mutations used to eliminate expression from the Cprime and C protein start codons are shown. Numbers refer to positions from the 5' end of the mRNA, and to the first base of the start codon.

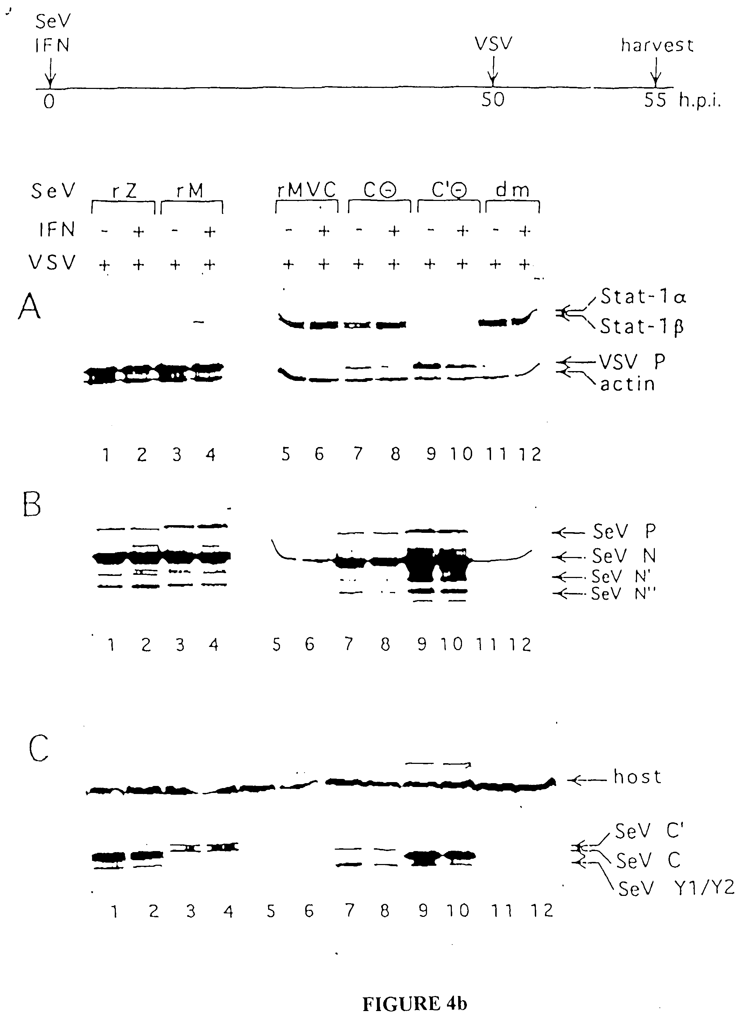

**Figure 4B :** Parallel cultures of BF cells were treated (or not) with 100 U of IFN-β, and infected at the same time with 5 pfu/cell of either rSeV^{Z}-C^{Z} (rZ, lanes 1,2), rSeV^{Z}-C^{M} (rM, lanes 3,4), rSeV^{Z}-C^{MVC} (rMVC, lanes 5,6), rSeV^{Z}-[C-minus] (C-, lanes 7,8), rSeV^{Z}-[C'-minus] (C'-, lanes 9,10) or rSeV^{Z}-C'/C-minus] (dm, double mutant, lanes 11,12). The cultures were then superinfected with 50 pfu/cell of VSV at 50h post-SeV infection, as indicated above. All cultures were harvested at 55 hpi. Cytoplasmic extracts were prepared, and equal samples (2% of a dish) were separated by SDS-PAGE and immunoblotted with a mixture of anti-VSV P, anti-Stat1, and anti-actin antibodies (panel A), a mixture of anti-SeV P and anti-SeV N antibodies (panel B), or antibodies to the SeV C protein (panel C). In panel C, note that the CM proteins (lanes 3,4) migrate slightly slower than the C^{Z} proteins (lanes 1,2, and 7-10), even though they are of the same length. The C^{MVC} (lanes 5,6) and Cdm proteins (lanes 11, 12) are not detected in these samples because these viruses grow relatively poorly in BF cells (panel B). A time line of the experiment is shown above.

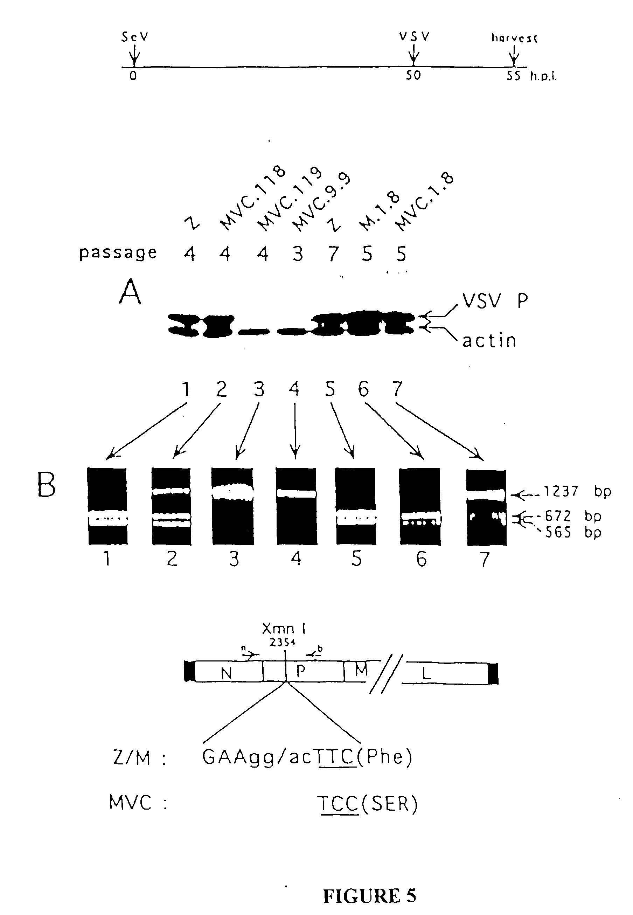

**Figure 5 :** Four independently generated rSeV^{Z}-C^{MVC} stocks, two of which continued to grow poorly in eggs (MVC.119 and MVC.9.9, lanes 3 and 4), and two of which began to grow normally in eggs (MVC.118 and MVC.1.8, lanes 2 and 7), along with two stocks of rSeV^{Z}-C^{Z} (lanes 1 and 5) and one of rSeV^{Z}-C^{M} (M1.8, lane 6), at the egg passage levels indicated, were used to infect BF cultures at a m.o.i. of 10-20. The cultures were superinfected with VSV at 50h post-SeV infection, and the relative amounts of VSV P protein intracellularly at 55 hpi was determined by immunoblotting (Panel A). RNA was isolated from these allantoic fluid stocks, and 1237 bp of the relevant region of the viral genome (T/C2354) was amplified by RT/PCR as shown below. The PCR product was digested with Xmn I, separated on an agarose gel, and stained with ethidium bromide (panel B).

### EXEMPLES :

### Exemple 1 : materials and methods

### 1.1. SeV stocks and infection

The generation of rSeV expressing alternate C (and P) proteins has been described (11, 12, 13, 22.). All SeV stocks were grown in the allantoic cavity of 10-day-old embryonated chicken eggs. Virus from allantoic fluid stocks were analyzed by SDS polyacrylamide gel electrophoresis and Coomassie blue staining after pelleting, and titers were determined by plaquing in LLC-MK2 cells.

The VSV stock (Mudd-Summers, Indiana) was grown in BHK cells. Virus released into the culture medium was clarified by centrifugation at 10,000 X g to remove cell debris, and the titer was determined by plaquing in LLC-MK2 cells. Experiments were performed by infecting (7 X 10⁶) mouse BF cells (36) with various SeV stocks at a multiplicity of infection (MOI) of 10-20 in a total volume of 3 ml of MEM plus 8% FCS, in the presence or absence of 100 U/ml of recombinant mouse (-Interferon (Calbiochem). At different times post-infection, cells were superinfected with VSV at a MOI of 50. Five hours later, cells were harvested, lysed in TNE plus 0.5 % NP-40. Proteins were analysed by immunoblotting, RNA was extracted with triazol (Gibco) and analysed by primer extension.

### 1.2. Immunoblotting

Proteins were separated by SDS-PAGE and tranferred to immobilon-P membranes by semi-dry transfer. The primary antibody antibodies used included:
- a rabbit polyclonal antiserum to VSV P protein (provided by J. Perrault and D. Summers),
- a rabbit polyclonal antiserum to SeV P protein isolated from a SDS gel (anti-PSDS, provided by L. Roux),
- a mouse monoclonal Ab to SeV N (N 877 (27)),
- a mouse monoclonal Ab to Stat1 (C-terminus) (Transduction laboratories, S21120),
- a rabbit polyclonal antiserum to actin (provided by G. Gabbiani, Geneva),
- a rabbit polyclonal antiserum to SeV C protein (provided by Y. Nagai, Tokyo).

The secondary antibodies used were AP-conjugated goat antibodies specific for either rabbit or mouse lgG (Biorad). The immobilized proteins were detected by light enhanced chemiluminescence (Biorad) and quantified using the Biorad fluor-S multimager.

### 1.3. Primer extension

Total intracellular RNA was isolated with Triazol and the genomes present were analyzed by primer extension, using MMLV reverse transcriptase and 200,000 cpm of 32P-5'-GAAGCTCCGCGGTACC-3', nucleotides 15,270-15,285, which had been purified on a sequencing gel.

### Exemple 2

As mentioned above, SeV^{MVC} was selected for its ability to replicate more efficiently than SeV^{M} in LLC-MK2 cells, and it was found to accumulate ca. 20-fold more viral protein and mRNA than SeV^{M}-infected cells (16, and data not shown). In BF cells, a cell line that is fully IFN-competent (i.e., secretes IFN in response to a viral infection, and responds to IFN by establishing an anti-viral state (36)), however, SeV^{M} actually grows slightly better (3 to 5-fold) than SeV^{MVC}, as estimated by the intracellular levels of viral proteins or genomes (lanes 1, 3, 4, fig 2A-D). To examine whether IFN might be involved in this reversed M/MVC replication efficiency, parallel BF cultures were pretreated with 100U of IFN-β for 24 h prior to SeV infection, and the accumulation of viral proteins and genomes was determined. SeV^{MVC} was found to be highly IFN-sensitive, as IFN pretreatment reduced the accumulation of viral products >20-fold, (lanes 2, 5 & 6, fig 2B and D). SeV^{M}, in contrast, was clearly less sensitive, as IFN pretreatment reduced the accumulation of viral products <5-fold (lanes 2, 5 & 6, fig 2A and C).

If endogenously generated IFN is involved in the reversed M/MVC replication efficiency in BF cells, infection with SeV^{MVC} should induce a general anti-viral state more strongly during this infection than infection with SeV^{M}. The rhabdovirus VSV is highly IFN-sensitive, and its inability to grow in cell culture is considered as a reliable indicator of this state. Some BF cultures were therefore pretreated with IFN-β for 24 h prior to M/MVC infection, and then superinfected with VSV at 48 hpi. We found that 48h of SeV^{MVC} infection were sufficient to prevent VSV proteins from accumulating during the VSV superinfection, even when these cultures had not been pretreated with IFN (lanes 3-6, fig 2F). In contrast, VSV proteins accumulated normally during the superinfection of cells infected with SeV^{M} for 48h (lanes 3 & 4, fig 2E). Thus, infection of BF cells with SeV^{MVC} induces an anti-VSV state (after 24-48h, data not shown), whereas infection with SeV^{M} does not induce this state, even at 72 hpi (data not shown). In all the experiments reported here, the appearance of normal levels of VSV P protein on superinfection correlated with strong cytopathic effects and cell death, whereas the absence of VSV P protein correlated with the absence of CPE and cell survival.

### Exemple 3 : SeVM infection, but not that of SeV^{MVC}, interferes with the IFN-mediated induction of an anti-viral state

The absence of an anti-VSV state following SeV^{M} infection could be because SeV^{M} avoids triggering the IFN system. Alternatively, SeV^{M} infection may interfere with the establishment of an anti-viral state, even though it does not suppress the anti-VSV state induced by 24h of IFN pretreatment (lanes 5 & 6, fig. 2E). BF cultures were therefore treated with IFN at the same time as they were infected, so that the IFN-mediated induction of the anti-viral state would take place concomitantly with the SeV infection, and not have a 24h head-start as in fig. 2. These cultures were then superinfected with VSV at 50h post-SeV infection, and the accumulation of VSV (and SeV) proteins was determined by immunoblotting at 55 hpi. The intracellular levels of the 91 kD Stat1α and 84 kD Stat1β proteins in these extracts were also determined by immunoblotting. Stat1 is a key component of the signaling cascade which ensues activation of the IFN receptor (6,9,26), and its elevated expression leads to programmed cell death (PCD), another cellular response to virus infection (3,20,29).

As shown in fig 3, 50h of infection with SeV^{M} (lanes 7,8) did not prevent the VSV P protein from accumulating normally (lane 3) on VSV superinfection, nor did it increase Stat1 levels over background. This infection, moreover, totally prevented IFN from inducing an anti-VSV state, as judged by the normal intracellular accumulation of the VSV P protein. In contrast, 50h of infection with SeV^{MVC} (lanes 9,10) strongly induced an anti-VSV state even in the absence of IFN treatment, and very dramatically increased Stat1 levels (up to 100-fold). SeV^{M} infection thus appears to interfere with the establishment of an IFN-mediated anti-viral state, as well as the elevated expression of Stat1.

### Exemple 4 : the role of the C protein

SeV^{M} and SeV^{MVC} differ by two aa substitutions, C^{F170S} and L^{E2050A} (fig. 1). To examine the role of the C gene mutation in suppressing the ability of SeV^{M} to interfere with the establishment of an IFN-mediated anti-viral state, we examined the infection of BF cells with rSeV^{Z}-C^{M} and rSeV^{Z}-C^{MVC}. These are chimeric viruses in which the resident C gene of SeV^{Z} (and the overlapping portion of the P gene) were replaced with that of SeV^{M} and SeV^{MVC} (the P genes of strains M and Z are 85% identical). rSeV^{Z}-C^{M} and rSeV^{Z}-C^{MVC} thus differ only by the C^{F170S} mutation. rSeV^{Z}-C^{Z}, in which the C gene was exchanged with itself as a cloning control (12), served as the wild-type rSeV^{Z}. As shown in fig 3, rSeV^{Z} (lanes 5,6) and rSeV^{Z}-C^{M} (lanes 11,12) behaved similarly to wild-type SeV^{M} (lanes 7,8). Fifty hours of these infections did not prevent the VSV P protein from accumulating normally on VSV superinfection, nor did it increase Stat1 levels over background. These infections also prevented IFN from inducing an anti-VSV state. In contrast, 50h of infection with rSeV^{Z}-C^{MVC} (lanes 13,14) strongly induced an anti-VSV state even in the absence of IFN treatment, but led to only a very modest increase in Stat1 levels over background (lane 13). This slight increase, however, is superior to that induced with IFN alone (lane 2), and IFN treatment plus rSeV^{Z}-C^{MVC} infection appeared to act synergistically on Stat1 levels (lane 14). Thus, viruses that contain a wild type C gene (SeV^{M}, rSeV^{Z}-C^{M}, or rSeV^{Z}-C^{Z}, all C^{F170}) appear to interfere efficiently with the establishment of an IFN-mediated anti-viral state, in contrast to those with a mutant C gene (SeV^{MVC}, rSeV^{Z}-C^{MVC}, both C^{S170}). The C proteins thus appear to be important in determining whether SeV infection will induce an anti-viral state.

### Exemple 5 : The AUG¹¹⁴-initiated C protein is specifically required to counteract IFN

The SeV C proteins are expressed as a nested set of four proteins (C^{prime}, C, Y1, and Y2) initiated at different start codons by a variety of ribosomal gymnastics, including the use of non-AUG codons, leaky scanning, and shunting (5,23). We have previously described rSeV which selectively do not express either their C^{prime} , C, or Cprime and C proteins (double mutant, dm), due to mutation of their respective start codons. The phenotypes of the C^{prime}-minus and C-minus viruses were similar. They grew slightly less well than wt virus in eggs, and their infections of BHK cells over-accumulated viral macromolecules, consistent with the ability of either C^{prime} or C (but not Y1 and Y2) to inhibit viral RNA synthesis (4). In spite of these similar phenotypes in eggs and cell culture, C^{prime}-minus was as virulent as rSeV^{Z} for mice, whereas C-minus was avirulent. The double mutant, which in contrast to the single mutants was relatively non-cytopathic in cell culture (and displayed a small plaque phenotype), was also avirulent at the highest doses tested (22).

Fig. 4 shows the effect of infection with rSeV which do not selectively express their C^{prime} or C proteins on Stat1 levels and subsequent VSV superinfection, in comparison to rSeV^{Z}-C^{M} and rSeV^{Z}-C^{MVC}. Only C^{prime}-minus behaved like its wt control (rSeV^{Z})(lanes 1 & 2). Fifty hours of C^{prime}-minus infection (lanes 9 & 10) did not induce an anti-VSV state (lane 9), and moreover prevented the effects of IFN treatment as well (lane 10), and the levels of Stat1 proteins were not elevated over background (lanes 9 & 10). C-minus infection (lanes 7 & 8), in contrast, and the double mutant (dm) infection (lanes 11 & 12) to an even greater extent, did (at least partially) induce an anti-VSV state, and significantly elevated Stat1 levels over background. Thus, the AUG¹¹⁴-initiated C protein (but not C', Y1, or Y2) is preferentially required to prevent SeV infection from inducing the IFN system in cell culture, consistent with its specific requirement for sustained viral replication in the mouse respiratory tract (22).

### Exemple 6 : Suppression of the IFN-mediated anti-VSV state is dominant

Although SeV^{MVC} grows ca. 20-fold better than SeV^{M} in MK2 cells, this virus actually grows ca. 5-fold less well than SeV^{M} in eggs (as well as in BF cells), and these relative replication efficiencies also apply to rSeV^{Z}-C^{MVC} vs rSeV^{Z}-C^{M}. All our rSeV arc isolated in hens eggs, and generally stocks at the 2nd or 3rd passage level (p2 or p3) are used to infect cell cultures. Six rSeV^{Z}-C^{MVC} stocks were originally isolated from DNA independently, and all behaved as shown (fig. 3) and grew relatively poorly in eggs. On further passage, however, some rSeV^{Z}-C^{MVC} stocks clearly grew better (5-10 fold) than others. As the F170S mutation apparently confers reduced growth in eggs (and the loss of an Xmn I site), the relevant region of the viral genomes in two stocks which grew well (p4MVC-118 and p5MVC-1.8) and two which grew poorly (p4MVC-119 and p3MVC-9.9) was amplified and examined (fig. 5). At the same time, BF cells were infected with the same stocks and superinfected with VSV at 48hpi as before, to determine whether infection with these stocks continued to induce an anti-VSV state.

As shown in fig. 5, the two stocks that continued to grow poorly (lanes 3,4) contained genomes in which the Xmn I site remained absent (ser at position 170), and 48h of infection with these stocks continued to strongly prevent subsequent VSV growth. In contrast, the two stocks that grew well (lanes 2,7); a) contained genomes which had regained the Xmn I site and had therefore reverted to phe at position 170, as well as those in which this site was absent (170S), and b) these stocks had lost the ability to prevent VSV growth, like rSeV^{Z}-C^{M} (lane 6). Influenza viruses that cannot express their NS1 protein grow poorly in eggs because they are more sensitive to the egg's developing IFN system (10). If SeV were also sensitive to the egg's IFN system, there would be pressure to select for mutants with restored ability to suppress IFN action. As the S170F reversion requires only the single back-transition, it would be expected to be present after several passages in eggs.

The infections in fig 5 were carried out at 10-20 pfu/cell, and cultures infected with p4MVC-118 or p5MVC-1.8 would thus be co-infected with a mix of rSeV^{Z}-C^{MVC} and the revertant rSeV^{Z}-C^{M}. In both these mixed infections (and other reconstituted mixed infections, not shown), the phenotype of the revertant rSeV^{Z}-C^{M} was dominant over rSeV^{Z}-C^{MVC} and the anti-viral state was not induced (i.e., VSV replication was not prevented by the rSeV^{Z}-C^{MVC} co-infection). The dominant nature of the suppression of the IFN-mediated anti-VSV state in mixed infections is consistent with this suppression being an active process.

To sustain its infection of the mouse respiratory tract, SeV must avoid or counteract the innate immune response of its animal host, which includes natural killer cells, the IFN system, and programmed cell death (PCD). The most virulent virus strains, like SeV^{M}, must be extremely efficient at these countermeasures, as they require only ca. 40 pfu to kill half the mice inoculated intranasally. SeV^{MVC}, a mutant of SeV^{M} with only two aa substitutions (one in the C gene and the other in the catalytic subunit of the viral polymerise (L)), however, appears to be virtually defenseless against the innate immune system of the mouse, as lung titers increase only during the first day of infection, and virus is then quickly cleared (16). Moreover, similar results were found for mice infected with rSeV^{Z}-C^{MVC}, a chimeric Z strain virus containing the mutant SeV^{MVC} C gene, or rSeV^{Z}-[C-minus], that contains a wild-type C gene but does not express the AUG¹¹⁴-initiated C protein. The SeV C gene thus plays a critical role in infections of its animal host, presumably by counteracting innate immunity.

Itoh *et al* (17) have recently found that SeV^{M} infections do not induce PCD either in MK2 cells or the mouse respiratory tract, whereas SeV^{MVC} infections are highly apoptogenic in both cases. The present work has found that SeV^{M} infection interferes with the IFN-mediated induction of an anti-viral state, in strong contrast to SeV^{MVC}. It is possible that SeV^{M} infections counteract the induction of PCD and the anti-viral effects of IFN by separate pathways, but it is more likely that these effects are linked. Cells from mice lacking PKR (35) or the 2-5A-dependent RNase L (37) show defects in PCD, suggesting a role for these enzymes in virus-induced, IFN-mediated PCD. Furthermore, several viruses have recently been found to induce PCD via activation of the IFN system (31). The Stat1 proteins themselves also induce genes involved in PCD (3,20,29), and SeV^{MVC} infection may be highly apoptogenic because it induces high levels of Stat1.

The molecular basis of the anti-viral response to IFN is not fully understood. More than 50 genes are induced by IFN-α/β(, but only a few of these gene products display intrinsic anti-viral activity, namely, p68 protein kinase (PKR) and 2-5A oligoadenylate synthetase (both dependent on ds-RNA for activity, 18), certain Mx family proteins (reviewed in 30), and most recently, the promyleocytic leukemia (PML) protein (2). While IFN-treated cells are resistant to many different viruses, individual over-expression of these intrinsically anti-viral proteins confers resistance only against some viruses. Thus, over-expression of PKR or 2-5A synthetase confers resistance to the picornavirus EMCV but not to the rhabdovirus VSV, whereas over-expression of human MxA or PML proteins confers resistance to VSV but not to EMCV (2). The enormous selective pressures imposed by viruses have resulted in a rich and diverse set of anti-viral pathways, and the anti-viral state induced via the IFN system is thus suitably complex. We have used two criteria, the ability of VSV to replicate following a period of SeV infection, and the intracellular levels of the Stat1 proteins, as indicators of this cellular anti-viral state. We have found that two different mutations in the C gent, the F170S substitution, and the specific loss of AUG¹¹⁴-initiated C protein (although C^{prime} is normally expressed and the Y1/Y2 proteins are over-expressed), largely eliminate the ability of the mutant infections to prevent the IFN-mediated anti-viral state. The SeV C gene thus plays a critical role in this process. The E2050A mutation in the L gene of SeV^{MVC} also appears to be involved in the attenuation of this virus, as the phenotype of SeV^{MVC} is consistently stronger than that of rSeV^{Z}-C^{MVC}, especially in preventing the elevation of Stat1 proteins, thereby eliminating one pathway to the induction of PCD.

Didock *et al* (8a) have recently reported that SeV strain H (SeV^{H}, which is very similar to SeV^{Z}) circumvents the IFN response, and does so by interfering with the transcriptional activation of IFN-responsive genes. This study used only wild-type SeV^{H}, and another paramyxovirus, SV5. However, it directly examined IFN signaling in BF cells, using transient transfection of plasmids in which a luciferase reporter gene was placed under the control of an IFN-α/β -responsive promoter (or an IFN-β promoter), followed by virus infection. The IFN-α/β-responsive promoter was found to be strongly activated both in mock- and SV5-infectecl cells treated with IFN, whereas little or no activation of this promoter was observed in SeV-infected cells. In contrast, SeV was a stronger inducer of the IFN-β promoter than SV5. These authors concluded that the failure of SeV^{H}-infected cells to respond to the substantial levels of IFN-α/β they produce was due to the effectiveness of the SeV^{H}-induced block. SeV^{M} and SeV^{MVC} infections of human peripheral blood mononuclear cells (PBMCs) are found to produce equivalent (and substantial) amounts of IFN-α [3395 I.U./ml for M, and 3776 I.U./ml for MVC (D. Hober, Lille, personal communication)]. Thus, all these wild-type SeV (strains M, Z, and H) presumably counteract the IFN-induced anti-viral state by interfering with the activation of IFN stimulated genes.

The dominant nature of rSeV^{Z}-C^{M} versus rSeV^{Z}-C^{MVC} in co-infections of eggs (fig. 5, and BF cells, data not shown) suggests that the C gene actively interferes with the induction of the anti-viral state rather than simply avoiding the induction, although it might do this as well. We have not as yet examined whether the C gene products can act in BF cells independent of virus infection. The C proteins might well interact with directly (and suppress) some component of the signaling pathway leading to an anti-viral state, as many viruses are known to subvert the IFN system by a wide variety of mechanisms (30). The apparently enhancing effect of the polymerase LE2050A mutation in viruses with C^{F170S} (SeV^{MVC}), however, suggests that the C proteins act as well via viral RNA synthesis. C is well placed to do this. The linear paramyxovirus genomes and antigenomes contain promoters for RNA synthesis at their 3' ends. The C protein acts as all inhibitor of viral RNA synthesis in a promoter specific fashion (1,32), i.e., it predominantly inhibits antigenome and mRNA synthesis from the genomic promoter. This inhibition depends on the relative ratio of C protein to genomes, which increases dramatically during the early stages of infection, as C is essentially a non-structural protein.

The SeV V protein is also known to affect viral RNA synthesis (3a). However, in contrast to rSeV-[C-minus), infection of BF cells with two different rSeV which cannot edit their P gene mRNA and do not express V [(6A (7) and A5G3 (13a)] was similar to that of wild-type virus. These rSeV-[edit-minus] infections did not induce an anti-VSV state, and they continued to prevent IFN (added concomitant with the infection) from inducing an anti-VSV state (data not shown). The SeV V protein therefore does not appear to be important in counteracting IFN action, although it is also thought to function in counteracting some aspect of innate immunity (19). The SeV C and V proteins have been referred to as "accessory" proteins, in the sense that they arc not expressed by all paramyxoviruses. For example, human parainflucnza virus type 1, the virus closest to SeV and a very successful parasite of children, does not contain a V ORF at all (25), and rubulaviruses (except for Newcastle disease virus) do not contain a C ORF. The overlapping C and V ORFs appear to have been added to the P ORF as a late event of virus evolution, possibly to adapt these viruses to a particular ecological niche, e.g., the mouse respiratory tract. The V gene clearly fits this description, providing a "luxury" function (19), as rSeV which do not express V are perfectly competent for growth in cell culture or eggs, and are only debilitated for growth in the mouse respiratory tract (7,8,19).

The C gene is more complex than the V gene. There are four independently initiated C proteins, and the phenotypes of viruses in which C^{prime} and C have been specifically ablated suggests that the various C proteins must, at least in part, have non-overlapping functions. While rSeV which cannot individually express C^{prime} or C grow well in (IFN-incompetent) BHK cells and accumulate viral macromolecules more rapidly than wild-type virus, the double mutant (which cannot express either C^{prime} or C) grows poorly in BHK cells, and accumulates viral macromolecules with delayed kinetics relative to wild-type virus. C^{prime} and C (but not Y1 or Y2) may provide a common function that accelerates the accumulation of viral products intracellularly, so that only the double mutant shows the loss of function phenotype (22). C^{prime} and C also appear to have non-overlapping functions, because although C or Y1/Y2 can replace C^{prime} for virulence in mice, C^{prime} or Y1/Y2 cannot replace C for this property (22). Y1 and Y2 presumably also play a role in intracellular virus replication, as rSeV which cannot express any of their C proteins have not been isolated (22), and those which at best express only some Y2 protein are at the limit of recovery from DNA (21,23). Unlike the V gene, the SeV C gene clearly plays an essential role in intracellular virus replication, even in the protected environment of defenseless cells in culture. What may have started out as a luxury function could have evolved to carry out essential functions in virus replication. Multiple C protein expression may then have evolved to better regulate their function.

The inventors have studied the relationship between the Sendai virus (SeV) C proteins (a nested set of four proteins initiated at different start codons) and the interferon-mediated antiviral response in IFN-competent cells in culture. SeV containing wild-type or various mutant C proteins were examined for; a) their ability to induce an antiviral state (i.e., to prevent the growth of VSV following a period of SeV infection), b) to induce the elevation of Stat1 protein levels, and c) to prevent IFN added concomitant with the SeV infection from inducing an antiviral state. The inventors find that expression of the wild-type C gene, and specifically the AUG¹¹⁴-initiated C protein, prevents the establishment of an antiviral state, i.e., cells infected with wild-type SeV exhibited little or no increase in Stat1 levels, and were permissive for VSV replication, even in the presence of exogenous IFN. In contrast, in cells infected with SeV lacking the AUG¹¹⁴-initiated C protein, or containing a single amino acid substitution in the C protein, the level of Stat1 increased and VSV replication was inhibited. The prevention of the cellular IFN-mediated antiviral response appears to be a key determinant of SeV pathogenicity.

### REFERENCES

1. Cadd, T., D. Garcin, C. Tapparel, M. Itoh, M. Homma, L. Roux, J. Curran and D. Kolakofsky. 1996. The Sendai Paramyxovirus Accessory C Proteins Inhibit Viral Genome Amplification in a Promoter-Specific Fashion. J. Virol., 70;5067-5074.
2. Chelbi-Alix, M.K., F. Quignon, L. Pelicano, M.H. Koken, and H. de Thé. 1998. Resistance to Virus Infection Conferred By The Interferon-induced Promyelocytic Leukemia Protein. J. Virol. 72:1043-1051.
3. Chin, Y.E., M. Kitagawa, K. Kuida, R.A. Flavell, and X.-Y. Fu. 1997. Activation Of The STAT Signaling Pathway Can Cause Expression Of Caspase 1 and Apoptosis. Mol. Cell. Biol. 17: 5328-5337.
3a. Curran, J., R. Boeck, and D. Kolakofsky. 1991. The Sendai virus P gene expresses both an essential protein and an inhibitor of RNA synthesis by shuffling modules via mRNA editing. EMBO J. 10:3079-3085.
4. Curran, J., J.-B. Marq, and D. Kolakofsky. 1992. The Sendai virus nonstructural C proteins specifically inhibit viral mRNA synthesis. Virology 189:647-656.
5. Curran, J., P. Latorre, and D. Kolakofsky. 1998. Translational Gymnastics on the Sendai virus P/C mRNA. Seminars in Virology, 8; 351-357.
6. Darnell, J.E. Jr. 1997. STATs And Gene Regulation. Science 277:1630-1635.
7. Delenda. C., S. Hausmann, D. Garcin, and D. Kolakofsky. 1997. Normal cellular replication of Sendai virus without trans-frame nonstructural V protein. Virology, 228; 55-62.
8. Delenda, C., G. Taylor, S. Hausmann, D. Garcin, and D. Kolakofsky. 1998. Sendai viruses with altered P, V, and W protein expression. Virology, 242; 327-37
8a. Didcock L, Young DF, Goodbourn S, and Randall RE. 1999. Sendai virus and simian virus 5 block activation of interferon-responsive genes: importance for virus pathogenesis. J Virol. 73:3125-33
9. Durbin, J.E., R. Hackenmiller, M.C. Simon, and D.E. Levy. 1996. Targeted Disruption Of The Mouse Stat1 Gene Results In Compromised Innate Immunity To Viral Disease. Cell 84:443-450.
10. Garcia-Sastre A, Egorov A, Matassov D, Brandt S, Levy DE, Durbin JE, Palese P, Muster T. 1998. Influenza A virus lacking the NS1 gene replicates in interferon-deficient systems. Virology 252:324-30
11. Garcin, D., T. Pelet, P. Calain, L. Roux, J. Curran, and D. Kolakofsky. 1995. A highly recombinogenic system for the recovery of infectious Sendai paramyxovirus from cDNA: generation of a novel copyback nondefective interfering virus. EMBO J., 14, 6087-6094.
12. Garcin, D., Itoh, M., and Kolakofsky, D. 1997. A point mutation in the Sendai virus accessory C proteins attenuates virulence for mice, but not virus growth in cell culture. Virology, 238; 424-431.
13. Garcin, D., Taylor G, Tanebayashi K, Compans R, and D. Kolakofsky. 1998. The short sendai virus leader region controls induction of programmed cell death. Virology. 243: 340-353
13a. Hausmann S, Garcin D, Morel AS, and Kolakofsky D. 1999. Two nucleotides immediately upstream of the essential A6G3 slippery sequence modulate the pattern of G insertions during Sendai virus mRNA editing. J. Virol. 73:343-51
14. Isaacs, A., and J. Lindenmann. 1957. Virus Interference. 1. The Interferon. Proc. R. Soc. Lond. (Biol.( 147:258-267.
15. Ishida, N., and M. Homma. 1978. Sendai Virus. Adv. Virus Res. 23:349-383.
16. Itoh, M., Y. Isegawa, H, Hotta, and M. Homma. 1997. Isolation of an avirulent mutant of Sendai virus with two amino acid mutations from a highly virulent field strain through adaptation to LLC-MK2 cells. J Gen Virol, 78, 3207-3215.
17. Itoh, M., H. Hotta, and M. Homma. 1998. Increased Induction Of Apoptosis By A Sendai Virus Mutant Is Associated With Attenuation Of Mouse Pathogenicity. J. Virol. 72: 2927-2934.
18. Jacobs, B.L., and J.O. Langland. 1996. When Two Strands Are Better Than One: The Mediators and Modulators of the Cellular Responses to Double-Stranded RNA. Virology 219:339-349.
19. Kato, A., K. Kiyotani, Y. Sakai, T. Yoshida, and Y. Nagai. 1997. The paramyxovirus Sendai virus V protein encodes a luxury function required for viral pathogenesis. EMBO J, 16; 678-587.
20. Kumar, A., M. Commane, T.W. Flickinger, C.M. Horvath, and G.R. Stark. 1997. Defective TNF(-Induced Apoptosis in STAT1-Null Cells Due To Low Constitutive Levels Of Caspases. Science 278:1630-1632.
21. Kurotani, A., K. Kiyotani, A. Kato, T. Shioda, Y. Saki, K. Mizumoto, T. Yoshida, and Y. Nagai. 1998 The paramyxovirus, Sendai virus, C proteins are categorically nonessential gene products but silencing their expression severely impairs viral replication and pathogenesis. Genes to Cells, 2:111-24..
22. Latorre, P., T. Cadd, M. Itoh, J. Curran, and D. Kolakofsky. 1998. The Various Sendai Virus C Proteins Are Not Functionally Equivalent And Exert Both Positive And Negative Effects On Viral RNA Accumulation During The Course Of Infection. J. Virol. 72:5984-5993.
23. Latorre, P., D. Kolakofsky, and J. Curran. 1998. Sendai Virus Y Proteins Are Initiated By A Ribosomal Shunt. Mol. Cell. Biol. 18: 5021-5031.
24. Marie, I., J.E. Durbin, and D.E. Levy. 1998. Differential Viral Induction Of Distinct Interferon-alpha Genes By Positive Feedback Through Interferon Regulatory Factor-7. Embo J. 17:6660-6669.
25. Matsuoka Y, Curran J, Pelet T, kolakofsky D, Ray R, Compans RW. 1991- The P gene of human parainflucnza virus type 1 encodes P and C proteins but not a cysteine-rich V protein. J Virol 1991 65:3406-10
26. Meraz, M.A., J.M. White, K.C. Sheehan, E.A. Bach, S.J. Rodig, A.S. Dighe, D.H. Kaplan, J.K. Riley, A.C. Greenlund, D. Campbell, K. Carver-Moore, R.N. DuBois, R. Clarks, M. Aguet, and R.D. Schreiber. 1996. Targeted Disruption of The Stat1 Gene In Mice Reveals Unexpected Physiologic Specificity In The JAK-STAT Signaling Pathway. Cell 84:431-442.
27. Orvell, C., and Grandien, M. 1982. The effects of monoclonal antibodies on biological activities of structural proteins of Sendai virus. J. Immunol. 129, 2779-2787.
28. Pelet T., Delenda C., Gubbay O., Garcin D., & Kolakofsky D. 1996. Partial characterization of a Sendai virus replication promoter and the rule of six. Virology 224:405-414.
29. Schindler, C. 1998. STATs As Activators Of Apoptosis. Trends Cell. Biol. 8: 97-98.
30. Stark, G.R., I.M. Kerr, B.R. Williams, R.H. Silverman, and R.D. Schreiber. 1998. How Cells Respond To interferons. Annu. Rev. Biochem. 67:227-264.
31. Tanaka, N., M. Sato, M.S. Lamphier, H. Nozawa, E. Oda, S. Noguchi, R.D. Schreiber, Y. Tsujimoto, and T. Taniguchi. 1998. Type I Interferons Are Essential Mediators Of Apoptotic Death In Virally Infected Cells. Genes Cells 3:29-37
32. Tapparel C., S. Hausmann, T. Pelet, J. Curran, D. Kolakofsky, & L. Roux. 1997. Inhibition of Sendai virus genome replication due to promoter-increased selectivity: a possible role for the accessory C proteins. J. Virol., 71: 9588-9599..
33. Vilcek, J., and Sen, G.C. 1996. Interferons and other cytokines. In Fields B.N., Knipe D.N., Howley P.M. (eds): Fields Virology, 3rd ed. Philadelphia: Lippincott-Raven, pp. 375-399.
34. Wathelet, M.G., C.H. Lin, B.S. Parekh, L.V. Ronco, P.M. Howley and T. Maniatis. 1998. Virus Infection Induces The Assembly Of Coordinately Activated Transcription Factors On The IFN-beta Enhancer In Vivo. Mol. Cell 1:507-518.
35. Yang, Y.L., L.F. Rcis, J. Pavlovic, A. Aguzzi, R. Schafer, A. Kumar, B.R. Williams, M. Aguet, and C. Weissmann. 1995. Deficient Signaling In Mice Devoid of Double-stranded RNA-dependent Protein Kinase. EMBO J. 14, 6995-7006.
36. Young, D.F., L. Didcock, and R.E. Randall. 1997. Isolation Of Highly Fusogenic Variants Of Simian Virus 5 From Persistently Infected Cells That Produce And Response to Interferon. J. Virol. 71:9333-9342.
37. Zhou, A., J. Paranjape, T.L. Brown, H. Nie, S. Naik, B. Dong, A. Chang, B. Trapp, R. Fairchild, C. Colmenares, and R.H. Silverman. 1997. Interferon action And Apoptosis Are Defective In Mice devoid Of 2',5'-oligoadenylate-dependent Rnase L. EMBO J. 16:6355-6363.
38. Zinkernagel, R.M., and H. Hengartner. 1997. Antiviral Immunity. Immunol. Today 18:258-260.

## Claims

1. Method for selecting attenuated virus wherein the genome of said virus contains at least one mutation compared to a wild type virus genome in at least one non-structural protein encoding gene, said method comprising the steps of :
. infecting a eukaryotic host cell with said mutated virus, said host cell being permissive to said virus ;
. determining the host cell anti-viral response mediated by interferon to the viral infection ;
. selecting said virus that induce said interferon mediated response that is higher than the interferon-mediated anti-viral response developed by a host cell infected by said wild-type virus.

2. Method for selecting attenuated virus wherein the genome of said virus contains at least one mutation compared to the wild type virus in at least one non-structural protein encoding gene, said method comprising the steps of :
. transfecting an eukaryotic host cell with said genome, said host cell having all the elements required to replicate and transcribe the mutated genome and to facultatively regenerate viral particles ;
. determining the host cell antiviral response mediated by interferon;
. selecting said virus whose genome induce said antiviral response that is higher than the interferon-mediated antiviral response developed by a wild-type virus genome.

3. Method for selecting a nucleic acid sequence encoding for a mutated non-structural protein of a virus, wherein said protein is conferring to the virus an attenuated phenotype, said method comprising the steps of :
. transfecting a host cell with said nucleic acid sequence, said host cell having all the elements required to express said non-structural protein ;
. measuring the host cell interferon mediated response;
. selecting said nucleic acid sequence that induce said response that is higher than the interferon mediated response of a host cell transfected with a wild-type nucleic acid sequence coding the non-structural protein.

4. Method according to any one of claims 1 to 3 wherein said virus belongs to the group of negative single strand RNA enveloped virus.

5. Method according to claim 4 wherein said negative single strand RNA enveloped virus is selected in the group consisting of Paramyxoviridae, Orthomyxoviridac, Rhabdoviridae, Bunyaviridae, Arenaviridae.

6. Method according to claim 5 wherein said Paramyxoviridae is selected in the group consisting of Newcastle disease virus, measles virus, mumps virus, human respiratory syncitial virus (hRSV), bovine respiratory syncytial virus (bRSV), human para-influenza virus types 1, 2, 3, 4a and 4b (h-PIV), bovine para-influenza virus type 3 (b-PIV), hendra virus, canine distemper virus, rinderpest virus, simian virus 5, Sendaï virus.

7. Method according to claims 1 to 6 wherein said gene or said nucleic acid sequence are selected in the group consisting of paramyxovirus protein C and C' encoding genes, morbilivirus C gene, rubulavirus NS genes, pneumovirus NS genes, W gene of Newcastle disease virus.

8. Method according to claim 7 wherein said paramyxovirus protein C encoding gene is selected in the group consisting of human para-influenza virus type 1 C genes, human para-influenza virus type 3 C genes, measles virus C gene, canine distemper virus C gene, rinderpest virus C gene, hendra virus C genes, Sendaï virus C genes.

9. Method according to claim 7 wherein said paramyxovirus NS gene is selected in the group consisting of human respiratory syncytial virus NS1 gene, bovine respiratory syncytial virus NS1 gene, mumps virus NS2 gene, simian virus 5 NS2 gene, human para-influenza type 2 NS2 gene, human para-influenza type 4a NS2 gene, human para-influenza type 4b NS2 gene, human respiratory syncytial virus NS2 gene, bovine respiratory syncytial virus NS2 gene.

10. Method according to any one of claims 1 to 9 wherein said mutation into said gene or said nucleic acid sequence is a substitution, a deletion or an addition.

11. Method according to claim 10 wherein said mutation introduces at least one amino-acid change in the non-structural protein.

12. Method according to claims 10 and 11 wherein said mutation into the viral genome is selected in a group consisting of naturally occurring mutation, genetically engineered mutation, chemically induced mutation, physically induced mutation.

13. Method according to any one of claims 1 to 12 wherein the host cell response mediated by interferon is determined by at least one of the following means
(a) measure of the level of transcription of a gene, the transcription of which is inducible by interferon ;
(b) measure of expression of a protein, the expression of which is inducible by interferon ;
(c) measure of the viral proliferation.

14. Method according to claim 13 wherein said gene is composed of :
. a promoter containing at least one functional interferon stimulated response element (IRSE), said promoter being selected in the group consisting of the genes involved in interferon signal transduction pathway, more particularly the IFN-inducible gene 9-27 ;
. a coding sequence selected in a group consisting of a coding sequence naturally under the control of a promoter according to a) and a sequence encoding for reporter gene;
and wherein said promoter is operably linked to said coding sequence.

15. Method according to claim 14 wherein said reporter gene is selected in the group consisting of genes coding for enzymes such as luciferase, β-galactosidase, alkaline phosphatase and genes coding for luminescent proteins such as the green fluorescent protein (GFP).

16. Method according to any one of claims 1 to 15 wherein the host cell is selected in a group consisting of :
(i) a cell that is part of a living animal ;
(ii) a eukaryotic cell that is cultured *in vitro*.

17. Method according to claim 16 wherein said living animal is a rodent, more particularly a mouse.

18. Method according to claim 16 wherein said *in vitro* cultured cell is selected in the group consisting of :
(i) a murine cell, particularly BF cells
(ii) a human cell, particularly MRC5 cells.

19. Method according to anyone of claims 1 to 18 wherein interferon is further added to the culture medium of host cells.

20. Method according to claims 3 wherein said nucleic acid sequence is cloned into a recombinant expression vector that contains all the elements required for the expression of said non-structural protein.

21. Method according to claim 20 wherein said vector is a pTM-1 vector that carries T7 polymerase promoter and an encephalomyocarditis virus internal ribosomal entry site (EMCV IRES).

22. Kit for determining the host cell response mediated by interferon comprising at least a nucleic acid construct containing
. a promoter sequence containing at least one functional interferon stimulated response element (IRSE), said promoter being selected in the group consisting of the genes involved in interferon signal transduction pathway, more particularly the IFN-inducible gene 9-27 ;
. a coding sequence selected from a group consisting of a coding sequence naturally under the control of a promoter according to a) and a sequence encoding for a reporter gene;

23. Kit according to claim 22 further comprising a host cell selected in the group consisting of a murine cell, particularly BF cells, and a human cell, particularly MRC5 cells.

24. Method to identify a mutation able to confer an attenuated phenotype to a virus wherein said method is comprising the following steps:
- a) sequencing said gene encoding said non-structural protein of an attenuated virus selected by the method according to claims 1, 2 and 4 to 19, or sequencing said nucleic acid sequence encoding for a mutated non-structural protein selected by the method according to claim 3.
- b) comparing the sequence of a wild type virus to the sequence obtained in a);
- c) identifying the mutation or the mutations.

25. Method for the production of an attenuated virus comprising the step of introducing in the genome of a virus at least one mutation identified by the method according to claim 24.

26. Attenuated virus obtainable by the method according to the claims 1, 2, 4 to 19 and 25.

27. Use of an attenuated virus according to claim 26 as a drug.

28. Use of an attenuated virus according to claim 27 as a vaccine for the prophylactic and/or therapeutic treatment of viral infection.

29. Use of an attenuated virus according to claim 26 as a delivering vehicle for gene therapy.

30. Use of an attenuated virus according to claim 26 as a delivering vehicle for genes encoding proteins of vaccine interest other than those of the virus itself, particularly to generate bivalent or multivalent vaccines to treat or prevent human or veterinary diseases caused by infectious pathogens, specifically viruses, bacteria and parasites.

31. Use of an attenuated virus according to claim 26 as a delivering vehicle for genes encoding proteins of vaccine interest other than those of the virus itself, particularly to generate vaccines to treat or prevent human or veterinary non-infectious diseases, specifically cancer and auto-immune diseases.

32. Nucleic acid sequence obtainable by the method according to the claims 3, 20 and 21.

33. Use of a nucleic acid sequence according to claim 32 to produce an attenuated virus.

34. Non-structural protein encoded by the nucleic acid sequence of claim 32.
